# EUROPEAN PATENT SPECIFICATION

(11) **EP 0 560 322 B1**
(45) Date of publication and mention of the grant of the patent: **08.05.1996**
(21) Application number: 93103837.6
(22) Date of filing: 10.03.1993
(51) Int. Cl.: C11D 1/10, C11D 3/00

(54) **Detergent composition**
Reinigungsmittelzusammensetzungen
Compositions détergentes

(30) Priority: 11.03.1992 JP 52693/92
(43) Date of publication of application: 15.09.1993
(73) Proprietor: Ajinomoto Co., Inc., Tokyo 104 (JP)
(72) Inventor: Sano, Keigo,c/o Cent. Res. Lab. Ajinomoto Co., Kawasaki-shi,Kanagawa-ken (JP); Takano, Shinichic/o Cent. Res. Lab. Ajinomoto Co., Kawasaki-shi,Kanagawa-ken (JP)
(74) Representative: Strehl Schübel-Hopf Groening & Partner

(56) References cited:
- FR-A- 2 457 891
- GB-A- 1 463 719
- US-A- 3 506 576
- DATABASE WPIL Week 8346, Derwent Publications Ltd., London, GB; AN 83-816699
- DATABASE WPIL Week 8944, Derwent Publications Ltd., London, GB; AN 89-320489

## Description

### SPECIFICATION

A detergent composition containing a salt of an anionic surfactant with a basic amino acid and containing from 0.005 to 10 % by weight of a water-soluble reducing agent and/or from 0.005 to 10 % by weight of a chelating agent.

### Field of the Invention

The present invention relates to a detergent composition containing a salt of an anionic surfactant with a basic amino acid.

### Prior Art

An attempt has heretofore been made to incorporate a salt of an anionic surfactant with a basic amino acid into various detergent compositions, since the salt is not so stimulative to the skin or mucous membrane (for example, as proposed in Japanese Patent Application Laid-Open Nos. 56-53196 and 58-168695). However, detergent compositions containing a salt of an anionic surfactant with a basic amino acid are difficult to put to practical use because of their poor storage stability and the offensive odor resulting from the basic amino acid.

The object of the present invention is to stabilize a detergent composition containing a salt of an anionic surfactant with a basic amino acid and to provide an odorless detergent composition containing the same.

The present inventors earnestly investigated for the purpose of solving the preceding problems and, as a result, have found that a detergent composition which contains a salt of an anionic surfactant with a basic amino acid and which is free from the offensive odor resulting from the basic amino acid may be obtained by incorporating from 0.005 to 10 % by weight of a water-soluble reducing agent and/or from 0.005 to 10 % by weight of a chelating agent to the composition. On the basis of the finding, they have completed the present invention.

Specifically the present invention relates to a detergent composition containing a salt of an anionic surfactant with a basic amino acid and containing from 0.005 to 10 % by weight of a water-soluble reducing agent and/or from 0.005 to 10 % by weight of a chelating agent.

The basic amino acid as referred to herein is an amino acid having basic side chain(s), such as lysine, arginine, ornithine, hydroxylysine and histidine.

The anionic surfactant for use in the present invention is a surfactant having at least one active group of -COO-, -OS0₃-, -SO₃-; -OPO₃²⁻ and -O(O)P0₂-. It includes, for example, fatty acids, N-acylglutamic acids, polyoxyethylene higher alcohol sulfate esters, high alcohol sulfate esters, alkylbenzenesulfonic acids, polyoxyethylene higher alcohol phosphate esters, high alcohol phosphate esters, monoalkylphosphoric acids, N-acylsarcosines, N-acyltaurins, isethi- onic acid high fatty acid esters, and higher alcohol sulfosuccinate esters.

The salt of a basic amino acid and an anionic surfactant may be obtained with ease by blending a basic amino acid and an anionic surfactant in a suitable solvent or in the absence of a solvent.

The water-soluble reducing agent for use in the present invention includes, for example, sulfites, thiosulfates, phosphites, hypophosphites, ascorbic acid and its derivatives. These may be used singly or in combination of them.

The concentration of the water-soluble reducing agent in the composition of the present invention varies, depending upon the kind of the agent and the kind and amount of the salt of a basic amino acid and an anionic surfactant to be in the composition. It is from 0.005 to 10 % by weight ("% by weight" is hereinafter simply referred to as "%"), preferably from 0.01 to 5 %. If it is less than 0.005 %, the effect of the agent for stabilizing the composition would be insufficient. lf, however, it is more than 10 %, the detergency of the composition would unfavorably lower.

The chelating agentfor use in the present invention includes, for example, ethylenediaminetetraacetic acid (EDTA), ethyleneglycol-bis (2-aminoethylether) tetraacetic acid, 1-hydroxyethylidene-1,1-diphosphonic acid (HEDP), ethylenediamine-tetra(methylenephosphonic acid), tripolyphosphoric acid, hexametaphosphoric acid, dihydroxyethylglycine, gluconic acid, saccharic acid, tartaric acid, citric acid and salts of them. These may be used singly or in combination of them.

The concentration of the chelating agent in the composition of the present invention varies, depending upon the kind of the agent and the kind and amount of the salt of a basic amino acid and an anionic surfactant to be in the composition. It is from 0.005 to 10 %, preferably from 0.01 to 3 %. If it is less than 0.005 %, the effect of the agent for stabilizing the composition would be insufficient. lf, however, it is more than 10 %, the chelating agent would often cause stimulation of the skin unfavorably.

The detergent composition of the present invention may optionally contain any other surfactant(s) selected from, for example, nonionic surfactants, cationic surfactants and amphoteric surfactants, in combination with the anionic surfactant. In addition, it may further contain, if desired, any other conventional components for detergents, for example, a moisturizer such as propylene glycol, glycerin or sorbitol; a viscosity adjusting agent such as methyl cellulose or ethanol; a microbicide such as triclosan or trichlorocarbam; an anti-inflammatory agent such as sodium glycyrrhetinate or tocopherol acetate; an antiseptic such as methylparaben or butylparaben; as well as an antifoaming agent, a protective colloidal agent, an ultraviolet absorbent, a whitening agent, a perfume and a dye, each in such a range that would not detract from the effect of the effect of the present invention.

The detergent composition of the present invention may be formed into any preparation form for any ordinary use, such as solid, paste or liquid, by any ordinary method.

Since the detergent composition of the present invention is not so stimulative to the skin and mucous membrane and is free from the offensive odor of basic amino acids, it may be used favorably as a solid soap, a body shampoo, a hair shampoo and a kitchen detergent.

### Examples

The present invention will be explained in detail by way of the following examples.

Determination of the odor of the samples as prepared by the following examples was effected by the sensual test by panelists of 10 men and 10 women. For evaluating the samples in the test,@indicates no odor, O indicates almost no odor, A indicates a little odor, and x indicates an offensive odor.

### Example 1

A water-soluble reducing agent and a chelating agent as shown in Table 1 below were added to 50 g of 30 % aqueous solution of L-lysine salt of coconut oil fatty acid and stored at 40°C for one month, whereupon the odor of each sample composition was determined. The results obtained are shown in Table 1.

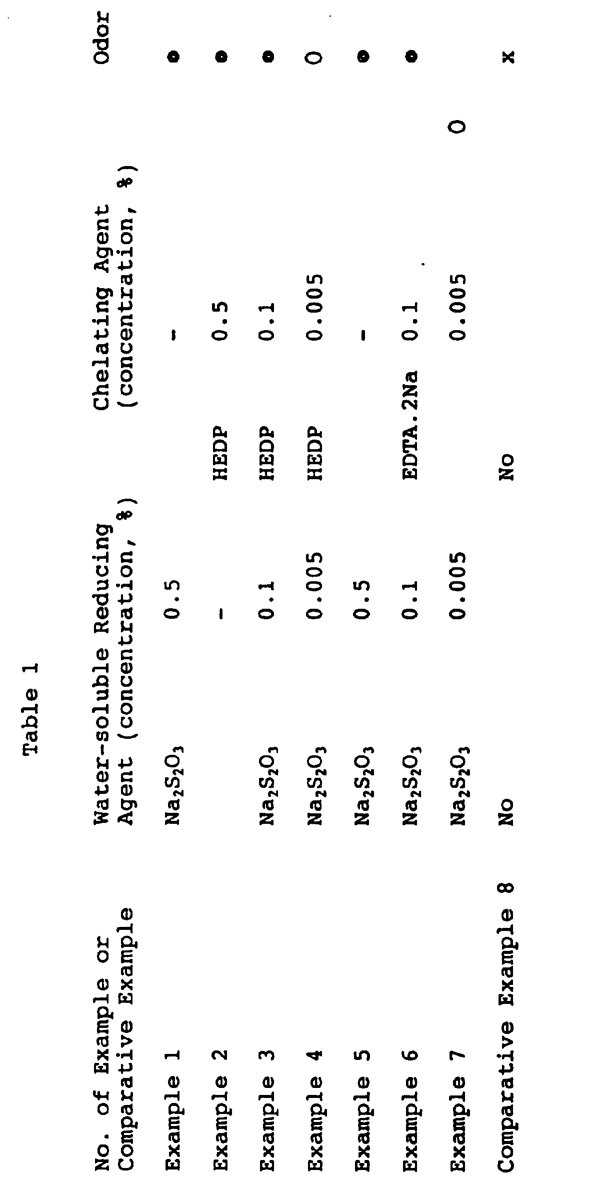

### Example 2

Water was added to 30 parts of a salt of an anionic surfactant with a basic amino acid as shown in Table 2 below and a water-soluble reducing agent (mixture of Na₂S₂O₃/Na₂SO₃ = 5/1) also shown in the same table to make 100 parts in all. They were dissolved and cooled to room temperature. The odor of each of the detergent compositions thus prepared was determined. The results are shown in Table 2.

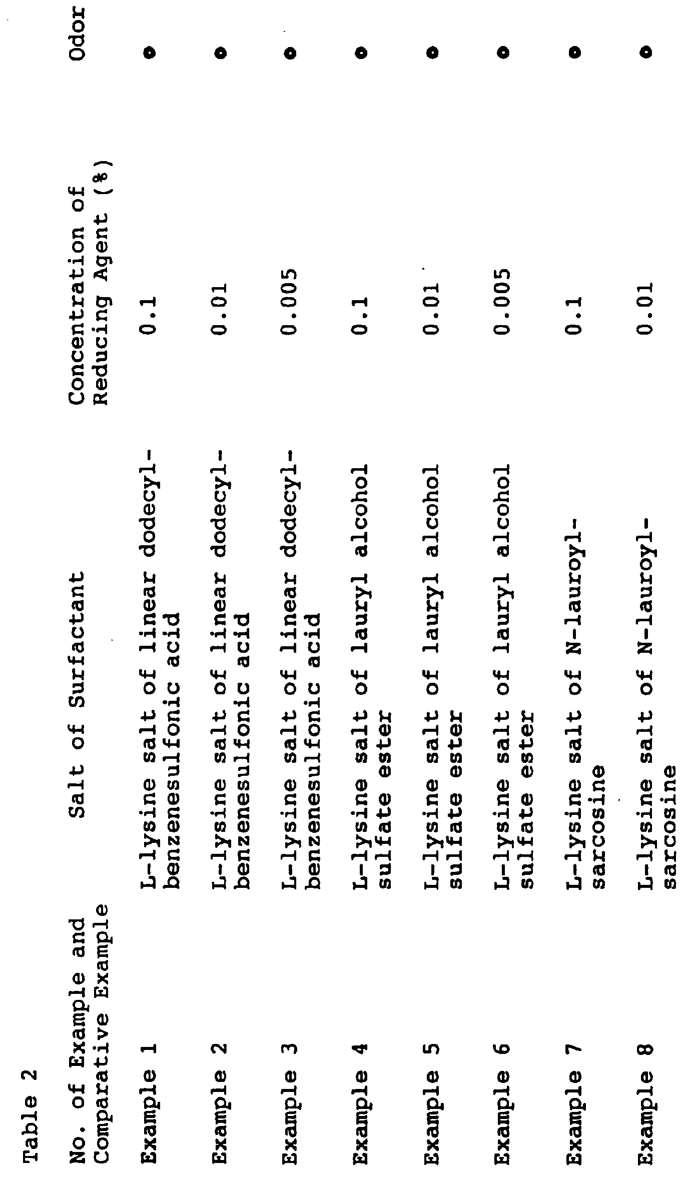

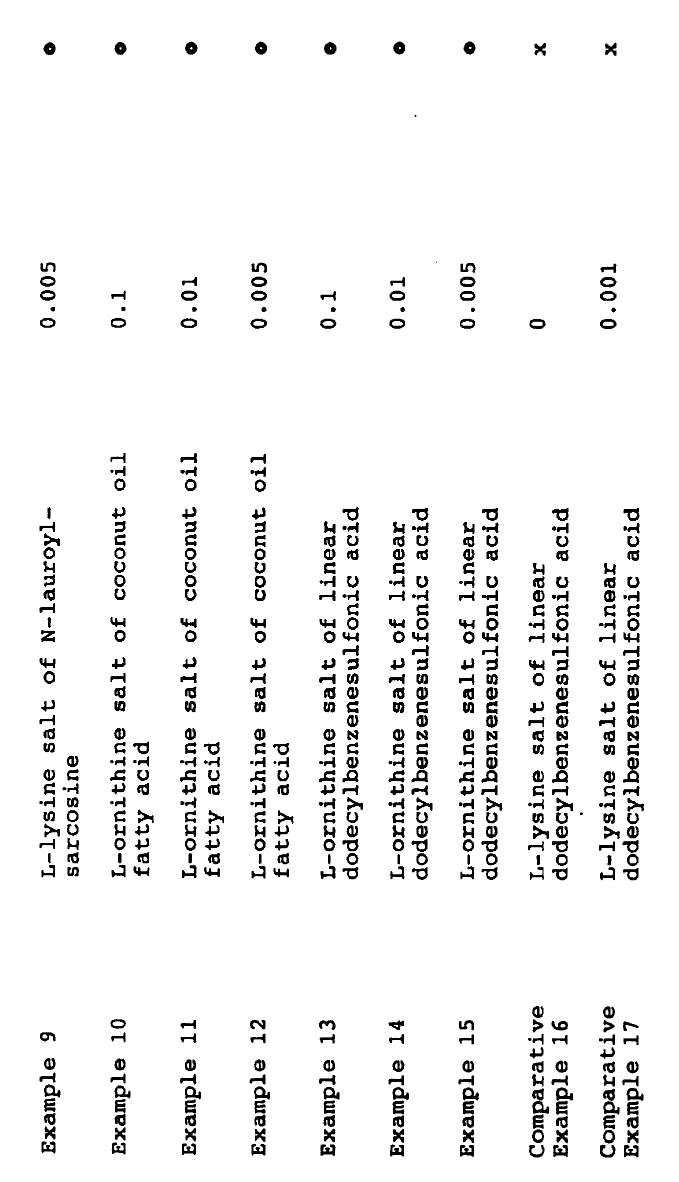

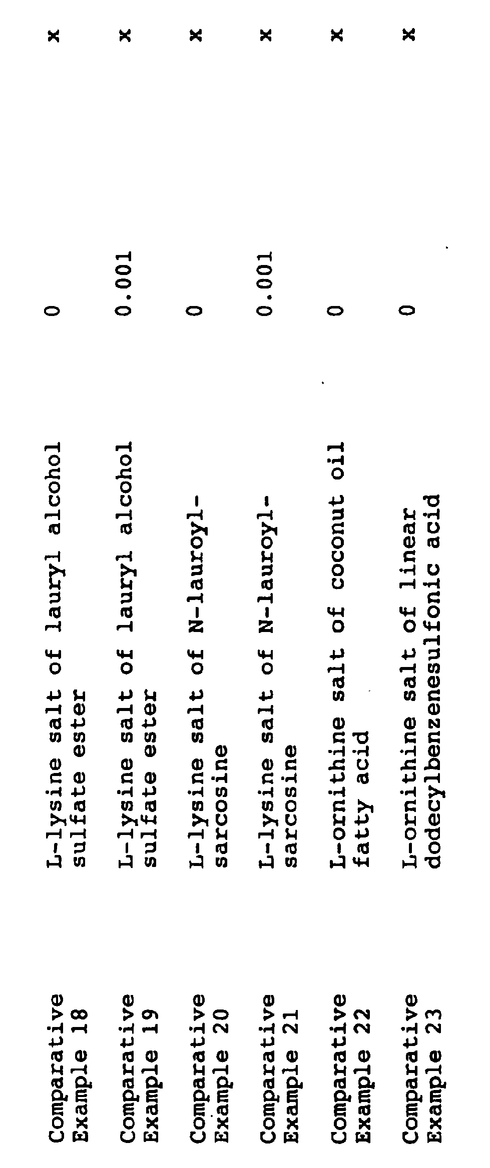

### Example 3: Face Wash Foam

### Components (A):

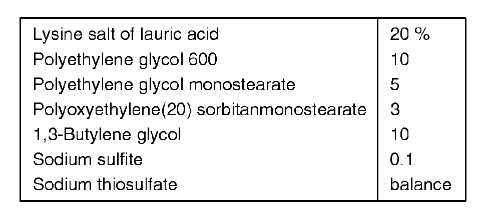

### Component (B):

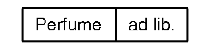

The components (A) were blended and dissolved at 80°C. The resulting blend was cooled with stirring, and the component (B) was added thereto at 60°C. After cooled to 30°C, a face wash foam was obtained. This was free from the offensive odor of the basic amino acid.

### Example 4: Solid Soap

### Component (A):

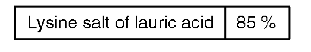

### Components (B):

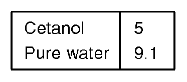

### Components (C):

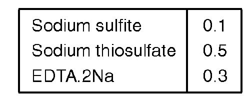

The components (B) were dissolved under heat at 80°C. The resulting blend was cooled with stirring, and the components (C) were added thereto at 60°C and cooled to 30°C. The component (A) was then added to the blend, uniformly kneaded with rolls and applied to an extruder. The extruded blend was shaped by stamping to obtain solid soaps. These were free from the offensive odor of the basic amino acid.

### Example 5: Solid Soap

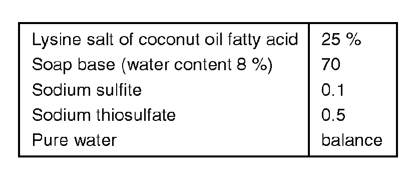

The aforesaid components were blended, uniformly kneaded with rolls and applied to an extruder. The extruded blend was shaped by stamping to obtain solid soaps. These were free from the offensive odor of the basic amino acid.

### Example 6: Solid Soap

### Components (A):

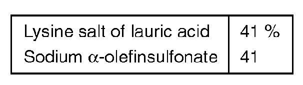

### Components (B):

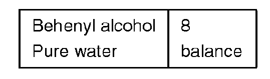

### Components (C):

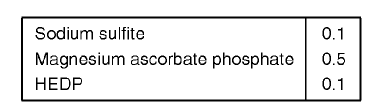

From the aforesaid components were obtained solid soaps in the same manner as in Example 4. These were free from the offensive odor of the basic amino acid.

### Example 7: Solid Soap

### Components (A):

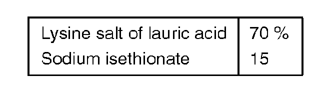

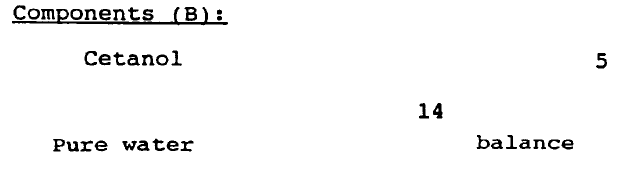

### Component (C):

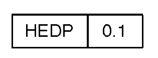

From the aforesaid components were obtained solid soaps in the same manner as in Example 4. These were free from the offensive odor of the basic amino acid.

### Example 8: Solid Soap

### Components (A):

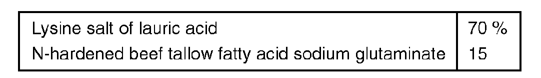

### Components (B):

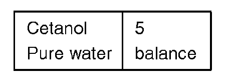

### Components (C):

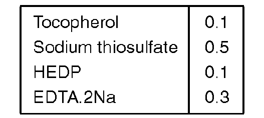

From the aforesaid components were obtained solid soaps in the same manner as in Example 4. These were free from the offensive odor of the basic amino acid.

### Example 9: Hair Shampoo

### Components (A):

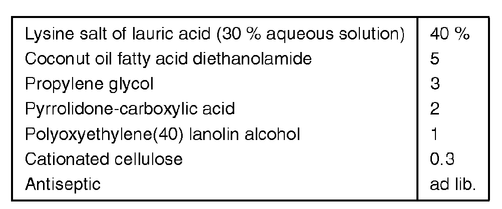

### Components (B):

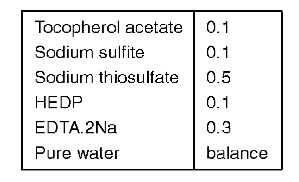

### Component (C):

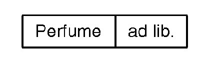

The components (A) and (C) were dissolved under heat at 80°C. The blend was cooled with stirring, and the component (C) was added thereto at 60°C. After cooled to 30°C, a hair shampoo-was obtained. This was free from the offensive odor of the basic amino acid.

### Example 10: Body Shampoo

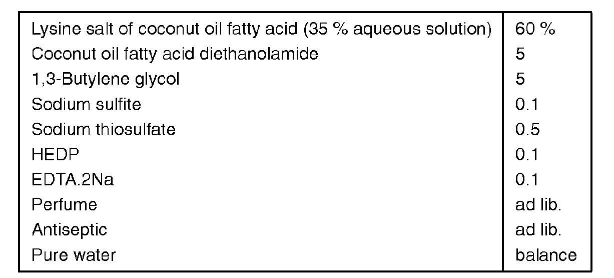

The body shampoo obtained from the aforesaid components was free from the offensive odor of the basic amino acid.

### Example 11: Kitchen Detergent

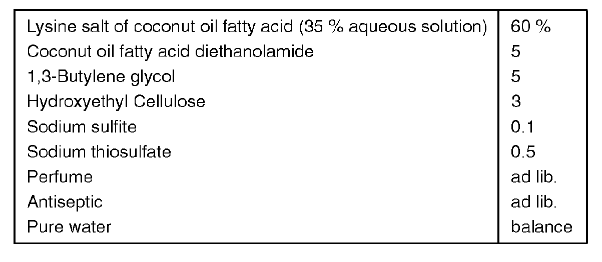

The kitchen detergent obtained the aforesaid components was free from the offensive odor of the basic amino acid.

## Claims (Claims for the following Contracting State(s) : DE, FR, GB)

1. A detergent composition containing a salt of an anionic surfactant with a basic amino acid and containing from 0.005 to 10 % by weight of a water-soluble reducing agent and/or from 0.005 to 10 % by weight of a chelating agent.

2. The detergent composition of claim 1, wherein said water-soluble reducing agent is selected from the group consisting of sulfites, thiosulfates, phosphites, hypophosphites and ascorbic acid.

3. The detergent composition of claim 1, wherein said water-soluble reducing agent is present in an amount of 0.01 to 5% by weight, based on the total weight of the composition.

4. The detergent composition of claim 1, wherein said chelating agent is selected from the group consisting of ethylenediaminetetraacetic acid, ethyleneglycol-bis(2-aminoethylether)tetraacetic acid, 1-hydroxyethylidene-1,1-diphosphonic acid, ethylenediaminetetra(methylenephosphonic acid), tripolyphosphoric acid, hexametaphosphoric acid, dihydroxyethylglycine, gluconic acid, saccharic acid, tartaric acid, citric acid and salts thereof.

5. The detergent composition of claim 1, wherein said chelating agent is present in an amount of 0.01 to 3 % by weight, based on the total weight of the composition.

## Claims (Claims for the following Contracting State(s) : ES)

1. A process for producing a stabilized detergent composition which comprising mixing a salt of an anionic surfactant with a basic amino acid with 0.005 to 10 % by wt. of a water-soluble reducing agent and/or from 0.005 to 10 % by wt. of a chelating agent

2. A process according to claim 1, wherein the salt of an anionic surfactant with a basic amino acid is obtained by blending a basic amino acid and an anionic surfactant in a suitable solvent or in the absence of a solvent.

3. A process according to claim 1 or claim 2, wherein the composition is further added with another surfactant.

4. A process according to any of the claims 1 to 3, wherein the stabilized detergent composition is prepared in the form of a solid, paste or liquid.

## Patentansprüche (Patentansprüche für folgende(n) Vertragsstaat(en) : DE, FR, GB)

1. Reinigungsmittelzusammensetzung, enthaltend ein Salz eines anionischen, oberflächenaktiven Mittels mit einer basischen Aminosäure und enthaltend 0,005 bis 10 Gew.-% eines wasserlöslichen Reduktionsmittels und/oder 0,005 bis 10 Gew.-% eines Chelatbildners.

2. Reinigungsmittelzusammensetzung nach Anspruch 1, wobei das wasserlösliche Reduktionsmittel aus der aus Sulfiten, Thiosulfaten, Phosphiten, Hypophosphiten und Ascorbinsäure bestehenden Gruppe ausgewählt ist.

3. Reinigungsmittelzusammensetzung nach Anspruch 1, wobei das wasserlösliche Reduktionsmittel in einer Menge von 0,01 bis 5 Gew.-%, bezogen auf das Gesamtgewicht der Zusammensetzung, vorliegt.

4. Reinigungsmittelzusammensetzung nach Anspruch 1, wobei der Chelatbildner aus der aus Ethylendiamintetraessigsäure, Ethylenglycol-bis-(2-aminoethylether)-tetraessigsäure, 1-Hydroxyethyliden-1,1-diphosphonsäure, Ethy- lendiamintetra(methylenphosphonsäure), Tripolyphosphorsäure, Hexametaphosphorsäure, Dihydroxyethylglycin, Gluconsäure, Zuckersäure, Weinsäure, Zitronensäure und Salzen dieser bestehenden Gruppe ausgewählt ist.

5. Reinigungsmittelzusammensetzung nach Anspruch 1, wobei der Chelatbildner in einer Menge von 0,01 bis 3 Gew- %, bezogen auf das Gesamtgewicht der Zusammensetzung, vorliegt.

## Patentansprüche (Patentansprüche für folgende(n) Vertragsstaat(en) : ES)

1. Verfahren zur Herstellung einer stabilisierten Reinigungsmittelzusammensetzung, das darin besteht, daß ein Salz eines anionischen oberflächenaktiven Mittels mit einer basischen Aminosäure mit 0,005 bis 10 Gew.-% eines wasserlöslichen Reduktionsmittels und/oder 0,005 bis 10 Gew.-% eines Chelatbildners vermischt wird.

2. Verfahren nach Anspruch 1, wobei das Salz eines anionischen oberflächenaktiven Mittels mit einer basischen Aminosäure durch Vermischen einer basischen Aminosäure und eines anionischen oberflächenaktiven Mittels in einem geeigneten Lösungsmittel oder in Abwesenheit eines Lösungsmittels erhalten wird.

3. Verfahren nach Anspruch 1 oder Anspruch 2, wobei der Zusammensetzung außerdem ein weiteres oberflächenaktives Mittel zugesetzt wird.

4. Verfahren nach einem der Ansprüche 1 bis 3, wobei die stabilisierte Reinigungsmittelzusammensetzung in Form eines Feststoffes, einer Paste oder einer Flüssigkeit zubereitet wird.

## Revendications (Revendications pour l'(les) Etat(s) contractant(s) suivant(s) : suivants : DE, FR, GB)

1. Composition de détergent contenant un sel d'un tensioactif anionique et d'un aminoacide basique contenant de 0,005 à 10% en poids d'agent réducteur hydrosoluble et/ou de 0,005 à 10% en poids d'un agent chélatant.

2. Composition de détergent selon la revendication 1, où ledit agent réducteur hydrosoluble est choisi dans le groupe constitué des sulfites, thiosulfates, phosphites, hypophosphites et acide ascorbique.

3. Composition de détergent selon la revendication 1, où ledit agent réducteur hydrosoluble est présent en une quantité de 0,01 à 5% en poids, par rapport au poids total de la composition.

4. Composition de détergent selon la revendication 1, où ledit agent chélatant est choisi dans le groupe constitué de l'acide éthylènediaminetétraacétique, l'acide éthylèneglycol-bis(2-aminoéthyléther)tétraacétique, l'acide 1-hydroxyéthylidène-1,1-diphosphonique, l'éthylènediaminetétra(acide méthylènephosphonique), l'acide tripoly- phosphorique, l'acide hexamétaphosphorique, la dihydroxyéthylglycine, l'acide gluconique, l'acide saccharique, l'acide tartrique, l'acide citrique et leurs sels.

5. Composition de détergent selon la revendication 1, où ledit agent chélatant est présent en une quantité de 0,01 à 3% en poids, par rapport au poids total de la composition.

## Revendications (Revendications pour l'(les) Etat(s) contractant(s) suivant(s) : suivant : ES)

1. Procédé de production d'une composition de détergent stabilisée qui comprend de mélanger un sel d'un tensioactif anionique et d'un aminoacide basique avec 0,005 à 10% en poids d'un agent réducteur hydrosoluble et/ou de 0,005 à 10% en poids d'un agent chélatant.

2. Procédé selon la revendication 1, où le sel d'un tensioactif anionique et d'un aminoacide basique est obtenu en mélangeant un aminoacide basique et un tensioactif anionique dans un solvant approprié ou en absence de solvant.

3. Procédé selon la revendication 1 ou 2, où la composition comprend l'addition d'un autre tensioactif.

4. Procédé selon l'une quelconque des revendications 1 à 3, où la composition de détergent stabilisée est préparée sous forme d'un solide, d'une pâte ou d'un liquide.
